# EUROPÄISCHE PATENTSCHRIFT

(11) **EP 0 476 503 B1**
(45) Veröffentlichungstag und Bekanntmachung des Hinweises auf die Patenterteilung: **02.08.1995**
(21) Anmeldenummer: 91115376.5
(22) Anmeldetag: 11.09.1991
(51) Int. Cl.: A61M 27/00

(54) **Saugdrainage zum Absaugen von Sekreten**
Suction drainage device for secretions suction
Dispositif de drainage par aspiration pour aspirer des sécrétions

(30) Priorität: 17.09.1990 DE 9013184 U
(43) Veröffentlichungstag der Anmeldung: 25.03.1992
(73) Patentinhaber: Wolter, Dietmar, Prof. Dr., D-21033 Hamburg (DE)
(72) Erfinder: Wolter, Dietmar, Prof. Dr., D-21033 Hamburg (DE)
(74) Vertreter: Glawe, Delfs, Moll & Partner

(56) Entgegenhaltungen:
- WO-A-89/05671
- DE-A- 3 533 369
- FR-A- 2 256 769
- US-A- 3 598 127

## Beschreibung

Die Erfindung betrifft eine Saugdrainage zum Absaugen von Sekreten und anderen Flüssigkeiten aus dem menschlichen oder tierischen Körper, die mit einem antibakteriell wirkenden Materialdepot und entlang ihrer Läugsausdehnung mit einer Vielzahl von Öffnungen versehen ist.

Es ist bekannt, daß durch Anwendung von Saugdrainagen, z.B. Redon-Saugdrainagen die Blutergußbildung und Gewebswasseransammlung in Operationswunden wesentlich vermindert werden kann. Dies führt zu einer besseren Wundheilung, da die Gewebeschichten sich aneinanderlegen können und dadurch direkt verheilen.

Bei jeder Operation kommt es trotz aller Maßnahmen zur Kontamination mit Keimen. In der Regel gelingt es den körpereigenen Abwehrkräften, die in die Wunde gelangten Keime abzutöten.

Begünstigender Faktor für die Ausbildung einer Infektion in der Operationswunde ist die Ansammlung von Sekret. Hier wirkt die herkömmliche Redon-Drainage durch die Sekretabsaugung prophylaktisch injektionsverhindernd. Bei infizierten Wunden besteht heute eine Therapiemöglichkeit darin, daß Polymethacrylatkugeln, die ein Antibiotikum enthalten, in die Wunde eingelegt werden. Durch die Diffusion des Antibiotikums kommt es im Verlauf von Tagen, Wochen und Monaten zur Abgabe des Antibiotikums in die Umgebung und damit zu einer wirksamen lokalen Keimbekämpfung. Es ist nicht nötig, den gesamten Körper z.B. durch orale Einnahme von Antibiotika zu belasten. Die Kugelketten haben aber den Nachteil, daß sie in der Regel operativ entfernt werden müssen, was bedeutet, daß sich der Patient einem zweiten Eingriff unterziehen muß.

Bei einer Saugdrainage der eingangs genannten Art ist es bekannt, ein antibakteriell wirkendes Materialdepot in einer semipermeablen Membran, insbesondere einem semipermeablen Schlauch einzuschließen, der dann mit der Saugdrainage verbunden wird, z.B. in diese Saugdrainage hineingesteckt wird. Dadurch erhält man aber einen verhältnismäßig komplizierten Aufbau der Saugdrainage. Außerdem ist bei den vorbekannten Ausführungsformen (DE-OS 35 06 288) unklar, wo und wie überhaupt abgesaugt wird. Es verhält sich offenbar so, daß vor allem in unmittelbarer Nähe des antibakteriell wirkenden Materialdepots abgesaugt wird, so daß die antibakteriell wirkenden Stoffe nur einen sehr kurzen Weg durch die Wunde zurücklegen. Außerdem ist die Lage des Materialdepots nicht genau definiert, da der semipermeable Schlauch offenbar sehr nachgiebig ist.

Es ist weiter bekannt, das Materialdepot am Ende des Saugschlauches innerhalb desselben in der Nähe von einer oder wenigen Öffnungen vorzusehen (WO89/05671). Dabei tritt aber eine medikamentöse Wirkung nur in der Nähe der Öffnung bzw. nur innerhalb des Schlauches auf, was bei einem Urinkatheter auch das eigentlich Wesentliche ist, da im wesentlichen nur unerwünschte Reaktionen innerhalb des Katheters vermieden werden müssen.

Es ist weiter bekannt, wesentliche Teile des Katheters mit antibakteriell wirkenden Materialdepots zu versehen (DE-A 35 33 369, US-A 3 598 127). Dabei kann dann aber wieder nicht dem Erfordernis Rechnung getragen werden, daß insbesondere bei der Wunddrainage am in den Körper einzubringenden Ende des Saugdrainageschlauches, dem distalen Ende dieses Schlauches eine größere antibakterielle Wirkung erforderlich ist als in weiter zum körperfernen Ende hin liegenden Bereichen des Schlauches.

Die Aufgabe der Erfindung besteht in der Schaffung einer Saugdrainage der eingangs genannten Art, die einerseits einfach aufgebaut und andererseits sehr wirksam ist.

Die erfindungsgemäße Lösung besteht darin, daß das Materialdepot das in den Körper einzubringende Ende des Saugschlauches abschließt. Das Materialdepot ist nicht nur wie beim Stand der Technik (DE-OS 35 06 288) mit dem Ende des Saugschlauches verbunden, so daß durch dieses Ende und den dort befindlichen semipermeablen Membranschlauch 3 Sekrete und gleichzeitig Wirkstoffe abgesaugt werden, bevor diese sich in der Wunde verteilen konnten. Vielmehr schließt das Materialdepot den Saugschlauch ab, so daß das antibakteriell wirkende Material hier leicht in den Körper eintreten und sich dort verbreiten kann, ohne daß es sofort abgesaugt wird. Es muß vielmehr mit den Sekreten und anderen Flüssigkeiten erst zu den Öffnungen des Saugschlauches gelangen, die in einem Abstand von seinem Ende angeordnet sind, und durch die Öffnungen abgesaugt werden. Auf diese Weise wird ein größerer Bereich der Wunde mit antibakteriell wirkenden Mitteln behandelt, wobei die Wirkung mit der Entfernung vom distalen Ende des Schlauchs abnimmt. Dies ergibt eine besonders vorteilhafte Dosierung entlang der Längsausdehnung des Schlauchs. Das antibakterielle Material muß auch immer erst einen gewissen Weg in der Wunde oder dergl. zurücklegen, bevor es dann mit dem Sekret abgesaugt wird.

Die besonderen Vorteile sind aber nicht nur bei Saugdrainagen gegeben, die in Operationswunden eingelegt werden. Ähnliche Vorteile treten vielmehr auch in allen anderen Fällen auf, bei denen ein Sekret aus dem menschlichen oder tierischen Körper abgesaugt werden muß. Als Beispiele seien hier noch die Torax-Drainage oder ein Blasenkatheter genannt, wenn sich Öffnungen nicht nur am Ende des Schlauches befinden sollen.

Bei einer vorteilhaften Ausführungsform ist das Materialdepot am in den Körper einzubringenden Ende des Saugschlauches innerhalb desselben angeordnet und dort vom Rest des Schlauches abgeteilt. Das Materialdepot befindet sich also in einer vorderen Kammer des Schlauches, die vom Rest des Schlauches z.B. durch einen Pfropfen abgeteilt ist. Das antibakteriell wirkende Material kann dann hier aus den Öffnungen des Schlauches herausdiffundieren und wird vom saugschlauch mit dem Sekret abgesaugt. Dabei legt das antibakteriell wirkende Material unterschiedlich große Wege zurück und tritt überall dort in den Drainageschlauch ein, wo dieser Öffnungen hat. Es wird also ein sehr großer Bereich der Operationswunde oder z.B. des Torax antibakteriell behandelt. Ähnliche Wirkungen werden erhalten, wenn das Materialdepot das in den Körper einzubringende Ende des Saugschlauches kappenförmig umgibt. Hier kann weiter vorgesehen werden, daß das kappenförmige Materialdepot lösbar am Ende des Saugschlauchs befestigt ist, so daß es im Körper verbleibt, wenn der Saugschlauch entfernt wird. Die antibakterielle Wirkung kann dann auch nach Entfernen des Saugschlauches weiter stattfinden, wobei das Materialdepot aus ausschließlich resorbierbaren Substanzen bestehen kann.

Zweckmäßigerweise nimmt die Größe der Öffnungen oder Löcher des Saugschlauchs in Saugrichtung ab, wie dies vom sogenannten "Ulmer Drain" bekannt ist. Dies hat den Vorteil, daß auch über größere Längen des Saugschlauches überall gleich stark abgesaugt wird, so daß auch das antibakteriell wirkende Material über größere Bereiche abgesaugt wird und damit über diese größeren Bereiche wirksam werden kann.

Bei einer anderen vorteilhaften Ausführungsform ist zusätzlich der Saugschlauch mit dem antibakteriell wirkenden Material beschichtet.

Zweckmäßigerweise ist das antibakteriell wirkende Material in ein vom Körper resorbierbares Material eingeschlossen. Dieses Material wird dann allmählich resorbiert, wobei dann auch das antibakteriell wirkende Material allmählich freigesetzt wird und über längere Zeiten wirksam sein kann.

Bei einer besonders vorteilhaften Ausführungsform besteht der Saugschlauch selbst wenigstens teilweise aus resorbierbarem Material, in dem das antibakteriell wirkende Material enthalten ist. Dadurch werden folgende wesentliche Vorteile erhalten.

Der Saugschlauch gibt im gesamten Bereich der Operationswunde das antibakteriell wirkende Material ab. Besonders kritisch für Keime ist normalerweise die Stelle, bei der der Saugschlauch durch die Haut austritt. Da hier an der Außenseite des Schlauchs ebenfalls antibakteriell wirkendes Material freigesetzt wird, ist an dieser Stelle die Keimbildung ebenfalls unterbunden. Eine weitere Möglichkeit besteht normalerweise darin, daß Keime entgegen der Saugrichtung durch das Sekret in den Körper zurückwandern. Dies wird ebenfalls verhindert, da der Saugschlauch überall antibakteriell wirkendes Material abgibt, so daß die Keime nicht in die Operationswunde oder den anderen Teil des Körpers zurückwandern können, in den der Saugschlauch eingelegt ist.

Zweckmäßigerweise ist das antibakteriell wirkende Material ein Antibiotikum, wobei besonders zweckmäßig Gentamycin ist.

Das resorbierbare Material kann Collagen, Polyglycolid-Lactid oder ein anderes geeignetes Polymer sein.

Insbesondere kann das antibakteriell wirkende Materialdepot Gentamycin und Polymethyl-Metacrylat als Trägersubstanz aufweisen. Durch unterschiedliche Mengenverhältnisse kann hier die Geschwindigkeit gesteuert werden, mit der das Antibiotikum freigesetzt wird. Diese Geschwindigkeit kann auch dadurch gesteuert werden, daß unterschiedliche Mengen eines Zusatzstoffes in das Material eingebaut werden, der den Abbau des Materialdepots und damit die Freisetzung des Antibiotikums beschleunigt oder verzögert. Die Geschwindigkeit der Freisetzung kann auch dadurch gesteuert werden, indem das Materialdepot unterschiedlich stark gepreßt wird oder z.B. schaumförmig aufgebaut wird.

Wenn der Schlauch aus resorbierbarem Material besteht, kann er eine Sollbruchstelle aufweisen, so daß ein Teil des Schlauches im Körper verbleiben kann, wenn die Absaugung abgeschlossen ist, um hier bis zur vollständigen Resorption keimtötend zu wirken.

Der Schlauch könnte auch ein Gefäßkatheter sein, der nicht nur zur Absaugung, sondern auch zum Infundieren dient.

Die Erfindung wird im folgenden anhand von vorteilhaften Ausführungsformen unter Bezugnahme auf die beigefügte Zeichnung beschrieben. Es zeigen:
- Fig. 1: im Längsschnitt eine erste Ausführungsform eines Saugschlauches, der für die Saugdrainage der Erfindung verwendet werden kann;
- Fig. 2: eine zweite Ausführungsform; und
- Fig. 3: eine dritte Ausführungsform.

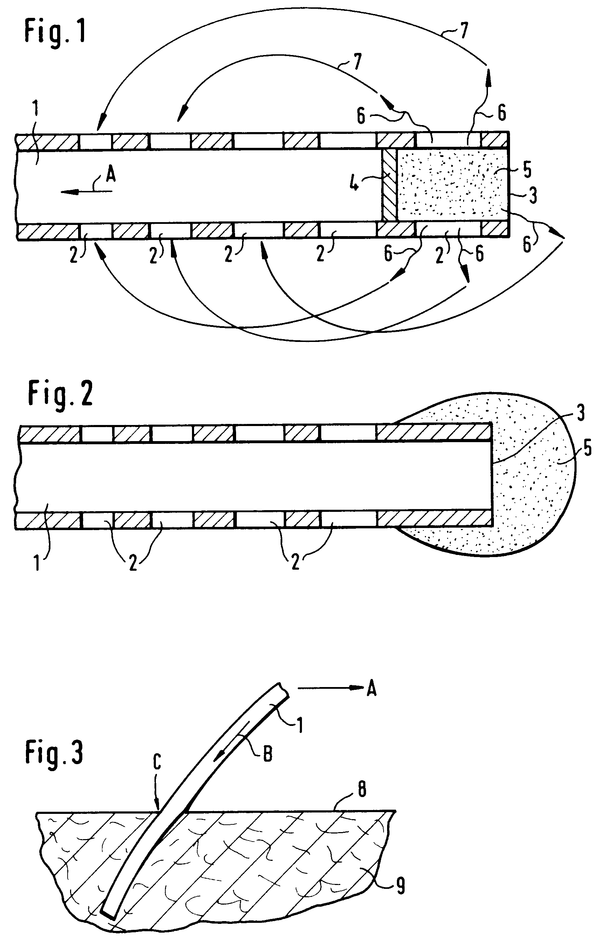

In Fig. 1 ist im Querschnitt ein Saugschlauch 1 gezeigt, der mit Öffnungen oder Löchern 2 versehen ist, durch die das Sekret abgesaugt werden kann. Der Saugschlauch 1 ist an eine Unterdruckquelle angeschlossen, so daß das Sekret in Richtung des Pfeiles A abgesaugt wird. In Saugrichtung nimmt dabei der Durchmesser der Öffnungen ab, wie dies in der Figur angedeutet ist. Durch einen solchen sogenannten "Ulmer Drain" wird erreicht, daß überall ungefähr die gleiche Saugwirkung stattfindet. Nahe seinem im Körper angeordneten Ende 3 ist der Schlauch 1 mit einem Pfropfen 4 abgeteilt, der z.B. angeschweißt ist. Zwischen dem Pfropfen 4 und dem Ende 3 befindet sich das Materialdepot 5, das das antibakteriell wirkende Material, z.B. ein Antibiotikum enthält. Dieses kann in vom Körper resorbierbares Material eingebettet sein. Das antibakteriell wirkende Material verläßt nun durch Diffusion den Schlauch 1 durch seitliche Öffnungen 2 und durch die Stirnöffnung am Ende 3, wie dies bei 6 angedeutet ist und legt längere Wege 7 durch die Wunde oder sonstige Körperhöhlung zurück, bis es durch die Öffnungen 2 eingesaugt wird. Im Gegensatz zum Stand der Technik wirkt also das antibakterielle Material über eine größere Strecke des Drainschlauches.

Bei der Ausführungsform der Fig. 2 ist das antibakterielle Material 5 als eine Kappe ausgebildet, die auf das Ende 3 des Schlauches 1 aufgesetzt ist. Die Wege 6, 7 des antibakteriell wirkenden Materials sind ähnlich wie in Fig. 1, so daß auf eine erneute Darstellung dieser Wege verzichtet wird. Wesentlich ist aber wieder, daß das Material bis zu allen Saugöffnungen 2 wandert und damit in größeren Bereichen der Körperhöhlung bzw. Wunde wirkt. Die Kappe 5 kann auch nur lose auf den Drainschlauch 1 aufgesetzt sein, so daß sie nach Entfernen des Drainschlauches 1 in der Wunde verbleibt. In diesem Fall sollte sie zweckmäßigerweise vollständig aus vom Körper resorbierbarem Material bestehen.

Bei der Ausführungsform der Fig. 3 besteht der gesamte Drainschlauch 1 aus einem resorbierbarem Material, das das antibakteriell wirkende Material enthält. Wird hier in Richtung des Pfeiles A abgesaugt, so läßt sich im allgemeinen doch nicht verhindern, daß sich Keime in Gegenrichtung, nämlich in Richtung B bewegen. Besteht aber der gesamte Schlauch aus resorbierendem Material mit antibakteriell wirkendem Material, so wird überall an den Schlauchwandungen antibakteriell wirkendes Material frei, so daß die Keime abgetötet werden.

Diese keimabtötende Wirkung besteht auch an der Stelle C, wo der Schlauch 1 durch die Haut 8 in das Gewebe 9 eintritt. Eine Infektion an der Stelle C wird dadurch wirksam verhindert. Selbstverständlich wird auch innerhalb des Körpers das Material des Schlauches 1 resorbiert und dabei das antibakteriell wirkende Material freigesetzt, so daß der Bereich der Wunde oder sonstigen Körperhöhlung antibakteriell behandelt wird.

## Patentansprüche

1. Saugdrainage zum Absaugen von Sekreten und anderen Flüssigkeiten aus dem menschlichen oder tierischen Körper, die mit einem antibakteriell wirkenden Materialdepot (5) und, entlang ihrer Längsausdehnung mit einer Vielzahl von Öffnungen (2) versehen ist, dadurch gekennzeichnet, daß das Materialdepot (5) das in den Körper einzubringende Ende des Saugschlauches (1) abschließt.

2. Saugdrainage nach Anspruch 1, dadurch gekennzeichnet, daß das Materialdepot (5) am in den Körper einzubringenden Ende (3) des Saugschlauches (1) innerhalb desselben angeordnet und dort vom Rest des Schlauches (1) abgeteilt ist.

3. Saugdrainage nach Anspruch 1, dadurch gekennzeichnet, daß das Materialdepot (5) das in den Körper einzubringende Ende des Saugschlauches (1) kappenförmig umgibt.

4. Saugdrainage nach Anspruch 3, dadurch gekennzeichnet, daß das kappenförmige Materialdepot (5) lösbar am Ende (3) des Saugschlauches (1) befestigt ist.

5. Saugdrainage nach einem der Ansprüche 2 bis 4, dadurch gekennzeichnet, daß die Größe der Öffnungen (2) des Saugschlauches (1) in Saugrichtung (A) abnimmt.

6. Saugdrainage nach einem der Ansprüche 1 bis 5, dadurch gekennzeichnet, daß der Saugschlauch (1) zusätzlich mit antibakteriell wirkendem Material (5) beschichtet ist.

7. Saugdrainage nach einem der Ansprüche 1 bis 6, dadurch gekennzeichnet, daß das antibakteriell wirkende Material (5) in ein vom Körper resorbierbares Material eingeschlossen ist.

8. Saugdrainage nach einem der Ansprüche 1 bis 7, dadurch gekennzeichnet, daß der Saugschlauch (1) wenigstens teilweise aus resorbierbarem Material besteht, in dem ebenfalls antibakteriell wirkendes Material (5) enthalten ist.

9. Saugdrainage nach einem der Ansprüche 7 oder 8, dadurch gekennzeichnet, daß das resorbierbare Material (5) Polyglycolid-Lactid ist.

10. Saugdrainage nach einem der Ansprüche 7 oder 8, dadurch gekennzeichnet, daß das resorbierbare Material (5) Collagen ist.

11. Saugdrainage nach einem der Ansprüche 8 bis 10, dadurch gekennzeichnet, daß der Saugschlauch (1) eine Sollbruchstelle aufweist.

12. Saugdrainage nach einem der Ansprüche 1 bis 11, dadurch gekennzeichnet, daß das antibakteriell wirkende Material (5) ein Antibiotikum ist.

13. Saugdrainage nach Anspruch 12, dadurch gekennzeichnet, daß das antibakteriell wirkende Material (5) Gentamycin ist.

14. Saugdrainage nach einem der Ansprüche 1 bis 13, dadurch gekennzeichnet, daß das antibakteriell wirkende Materialdepot (5) Polymethyl-Metacrylat als Trägersubstanz aufweist.

15. Saugdrainage nach einem der Ansprüche 1 bis 14, dadurch gekennzeichnet, daß das antibakteriell wirkende Materialdepot (5) einen den Abbau desselben beschleunigenden oder verzögernden Zusatzstoff aufweist.

## Claims

1. Suction drain for the aspiration of discharges and other fluids from the human or animal body, which is provided with a material depot (5) with an antibacterial action, and, along its longitudinal extent, with a plurality of openings (2), characterized in that the material depot (5) terminates the suction tube (1) end to be introduced into the body.

2. Suction drain according to Claim 1, characterized in that the material depot (5) is arranged at the suction tube (1) end (3) to be introduced into the body, in the interior of the tube (1) and there partitioned off from the remainder thereof.

3. Suction drain according to Claim 1, characterized in that the material depot (5) surrounds in the manner of a cap the suction tube (1) end to be introduced into the body.

4. Suction drain according to Claim 3, characterized in that the cap-like material depot (5) is releasably attached at the end (3) of the suction tube (1).

5. Suction drain according to any of Claims 2 to 4, characterized in that the size of the openings (2) in the suction tube (1) decreases in the direction of suction (A).

6. Suction drain according to any of Claims 1 to 5, characterized in that the suction tube (1) is additionally coated with material (5) with an antibacterial action.

7. Suction drain according to any of Claims 1 to 6, characterized in that the material (5) with an antibacterial action is enclosed in a material which can be absorbed by the body.

8. Suction drain according to any of Claims 1 to 7, characterized in that the suction tube (1) consists at least partially of absorbable material in which material (5) with an antibacterial action is also contained.

9. Suction drain according to either of Claims 7 or 8, characterized in that the absorbable material (5) is polyglycolide-lactide.

10. Suction drain according to either of Claims 7 or 8, characterized in that the absorbable material (5) is collagen.

11. Suction drain according to any of Claims 8 to 10, characterized in that the suction tube (1) has an intended breaking point.

12. Suction drain according to any of Claims 1 to 11, characterized in that the material (5) with an antibacterial action is an antibiotic.

13. Suction drain according to Claim 12, characterized in that the material (5) with an antibacterial action is gentamicin.

14. Suction drain according to any of Claims 1 to 13, characterized in that the material depot (5) with an antibacterial action has polymethyl-methacrylate as carrier substance.

15. Suction drain according to any of Claims 1 to 14, characterized in that the material depot (5) with an antibacterial action has an additive which speeds up or slows down the breakdown thereof.

## Revendications

1. Dispositif de drainage par aspiration pour aspirer des sécrétions et d'autres liquides à partir du corps humain ou animal, muni d'un dépôt (5) de matière à action antibactérienne et, le long de son étendue longitudinale, d'une multiplicité d'ouvertures (2), caractérisé en ce que le dépôt de matière (5) obture l'extrémité du tuyau flexible d'aspiration (1) destinée à être introduite dans le corps.

2. Dispositif de drainage par aspiration selon la revendication 1, caractérisé en ce que le dépôt de matière (5) est disposé à l'intérieur du tuyau flexible d'aspiration (1) à l'extrémité (3) de celui-ci qui doit être introduite dans le corps, et il y est séparé du reste du tuyau flexible (1).

3. Dispositif de drainage par aspiration selon la revendication 1, caractérisé en ce que le dépôt de matière (5) entoure à la manière d'une coiffe l'extrémité du tuyau flexible d'aspiration (1) qui doit être introduite dans le corps.

4. Dispositif de drainage par aspiration selon la revendication 3, caractérise en ce que le dépôt de matière (5) en forme de coiffe est fixé de façon détachable à l'extrémité (3) du tuyau flexible d'aspiration (1).

5. Dispositif de drainage par aspiration selon l'une quelconque des revendications 2 à 4, caractérisé en ce que la dimension des ouvertures (2) du tuyau flexible d'aspiration (1) décroît dans la direction d'aspiration (A).

6. Dispositif de drainage par aspiration selon l'une quelconque des revendications 1 à 5, caractérisé en ce que le tuyau flexible d'aspiration (1) est en plus revêtu de matière à action antibactérienne (5).

7. Dispositif de drainage par aspiration selon l'une quelconque des revendications 1 à 6, caractérisé en ce que la matière à action antibactérienne (5) est incluse dans une matière susceptible de résorption par le corps.

8. Dispositif de drainage par aspiration selon l'une quelconque des revendications 1 à 7, caractérisé en ce que le tuyau flexible d'aspiration (1) est fait, en partie au moins de matière résorbable dans laquelle est également contenue de la matière à action antibactérienne (5).

9. Dispositif de drainage par aspiration selon la revendication 7 ou 8, caractérisé en ce que la matière résorbable (5) est un polyglycolide-lactide.

10. Dispositif de drainage par aspiration selon la revendication 7 ou 8, caractérisé en ce que la matière résorbable (5) est du collagène.

11. Dispositif de drainage par aspiration selon l'une quelconque des revendications 8 à 10, caractérisé en ce que le tuyau flexible d'aspiration (1) présente un point destiné à la rupture.

12. Dispositif de drainage par aspiration selon l'une quelconque des revendications 1 à 11, caractérisé en ce que la matière à action antibactérienne (5) est un antibiotique.

13. Dispositif de drainage par aspiration selon la revendication 12, caractérisé en ce que la matière à action antibactérienne (5) est de la gentamicine.

14. Dispositif de drainage par aspiration selon l'une quelconque des revendications 1 à 13, caractérisé en ce que le dépôt de matière à action antibactérienne (5) comporte, en tant que substance de support, un polyméthacrylate de méthyle.

15. Dispositif de drainage par aspiration selon l'une quelconque des revendications 1 à 14, caractérisé en ce que le dépôt de matière à action antibactérienne (5) comporte un additif qui accélère ou ralentit sa dégradation.
